# EUROPEAN PATENT APPLICATION

(11) **EP 0 748 792 A1**
(43) Date of publication of application: **18.12.1996**
(21) Application number: 95912526.1
(22) Date of filing: 24.02.1995
(51) Int. Cl.: C07C 235/80, A61K 31/16

(54) **CALCIUM KETOPANTOTHENATE WITH ACETYLATING PROPERTIES**

(30) Priority: 02.03.1994 RU 94008016
(71) Applicant: TOVARISCHESTVO S OGRANICHENNOI OTVETSTVENNOSTJU " PANT", Moscow 117820 (RU)
(72) Inventor: KOZLOVA, Galina Sergeevna, Moscow, 117513 (RU); SEMINA, Olga Ivanovna, Moscow, 117485 (RU); GUNAR, Vladimir Ivanovich, Moscow, 117393 (RU); KRYLOV, Jury Fedorovich, Moscow, 123007 (RU)
(74) Representative: Desrousseaux, Grégoire Marie
(86) International application number: RU9500032
(87) International publication number: WO9523782

(57) **Abstract**

A novel compound, namely calcium ketopantothenate, is obtained by the condensation of ketopantolactone with a calcium salt of β-alanine in low aliphatic alcohols as solvent at a temperature of 65-70° C. It has been shown that calcium ketopantothenate has acetylating properties analogous to those of calcium D-pantothenate (vitamin B₃ or B₅), is of low toxicity and can be used as a vitamin agent in medicine, livestock breeding and cosmetics.

## Description

### Field of the invention

This invention relates to a novel chemical compound, the calcium salt of ketopantothenic acid (calcium ketopantothenate), as well as to its applications, based on the acetylating properties of the compound, manifested in the bodies of people and animals and allowing it to be considered as an analogue of a widely known vitamin agent (vitamin B₃ or B₅).

### Background of the invention

At present pantothenic acid is widely used in medicine as a prophylactic and therapeutic drug, and livestock breeding, especially in poultry and pig-breeding, as a vitamin fodder additive. Shortage of vitamins results growth retardation of the animals, decline in their reproduction and depression of their immunity to infectious diseases.

Pantothenic acid manifests the biological properties only in D-(+) form while its D,L-form is half as active. D-pantothenic acid in the organism is incorporated into the structure of Acetyl coenzyme A (Acetyl CoA), which plays the central role in the metabolism of men and animals. Being an acetylating coenzyme, CoA in the acetyl form takes part in the biosynthesis of carbohydrates, fatty acids, amino acids and proteins, stearines, in the synthesis of acetylcholine. Acetyl CoA is necessary for acetylating reactions in the process of biotransformation of alien toxic compounds in the organism in order to make them harmless and to eliminate them from the organism. Reaction with acetic acid is the main way of inactivation of a large number of medical compounds: sulfanilamides, hydrazides, p-aminobenzoic acid derivatives, etc.

CoA is formed in the organism with the participation of cysteine, adenosine triphosphate and pantothenic acid, the pantothenic acid is the only irreplaceable component in CoA biosynthesis.

Calcium D-pantothenate is produced for medical and the food industry, whereas calcium D, L-pantothenate, containing less than 50 % of active substance, is produced for fodder vitaminization.

The intermediate of calcium D,L-pantothenate synthesis is synthetic D,L-pantolactone. For calcium D-pantothenate synthesis, D-pantolactone, produced from D,L-pantolactone by different methods, is used.

All the known methods are multistage processes with high usage of the raw materials and energy and produce a large quantity of waste materials. The process of D-pantolactone production determines the economics of the vitamin production. The world market price of the vitamin in the D-form is three times higher than the price of the D,L-form.

### Summary of the invention

The goal of the invention is to obtain novel compounds with vitamin activity not less than calcium D-pantothenate activity, but with a lower production cost.

The authors obtained a calcium salt of ketopantothenic acid with acetylating properties, analogous to calcium D-pantothenate properties, and having a lower production cost in comparison with calcium D-pantothenate.

The illustrated compound was not published before, it was obtained by the authors for the first time. The ketopantothenic acid itself was described in the following publications:
1. Wieland, Chem. Ber., 81, 323 (1948),
2. Nagase O., Hosokawa Y., Shimizu M., Chem. Pharm. Bull., 17 (2), 398-9 (1969)
in particular in connection with the possibilities of the ketopantothenic acid usage in the process of microorganism cultivation.

The claimed compound is obtained by the condensation of ketopantolactone with a calcium salt of β-alanine in low aliphatic alcohols as solvent at a temperature of 30-90° C, preferably at 65-70° C, with further isolation of the reaction product by precipitation with acetone or evaporating and drying of an aqueous solution of the final product purified by extraction. The intermediate used in this process, namely ketopantolactone, is obtained by a well-known process of dehydrogenation of pantolactone, eliminating the expensive process of pantolactone separation into its enantiomers, which makes the production less expensive and ecologically safe.

The novel compound has the following chemical structure:

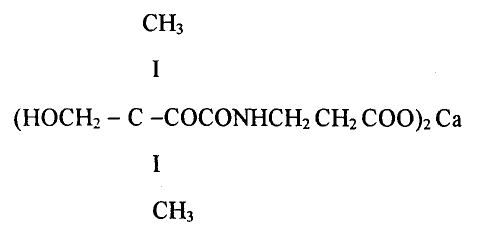

The calcium ketopantothenate structure has been proven with the help of the elemental analysis and infra-red spectrum data.

Calcium ketopantothenate is a white powder, readily soluble in water, less soluble in alcohol and sparingly soluble in acetone and chloroform.

### Examples of execution of the invention

### Synthesis of the claimed substance

### Example 1

1.02 g (0.025 g-mol) of metallic calcium is heated for 1 hour in 25 ml of absolute ethyl alcohol, then 4.46 g (0.05 g-mol) of β-alanine are added and mixed for 1 hour at a temperature of 65-70° C. 6.55 g (0.05 g-mol) of ketopantolactone are added to the obtained mass and mixed for 2 hours at a temperature of 65° C. Then the alcohol is distilled of, the residue is dissolved in 40 ml water and then extracted with chloroform. The aqueous solution is filtered, evaporated and dried. 10.2 g of final product are obtained. The output is 89.7 %. The melting point is 210-212° C.

| | | | | | |
|---|---|---|---|---|---|
| Found % | C 45.42 %, | H 6.05 %, | N 6.03 %, | Ca 8.52 %, | C₁₈H₂₈O₁₀N₂Ca |
| Calculated for % | C 45.47 %, | H 5.97 %, | N5.92%, | Ca 8.46 % | |

Infra-red spectrum cm⁻¹: 3400 (OH, NH), 1700 (CO), 1660, 1560 (amide I,II);

### Example 2

1.43 g of calcium oxide is added to 4.46 g of β-alanine in 25 ml of methanol and mixed for 1 hour at a temperature of 60-65° C. Then 6.55 g of ketopantolactone are added and mixed for 1 hour while heating. Then methanol is distilled off until a syrup-like residue is obtained and 50 ml of acetone is added with mixing. The residue is filtered , washed with acetone and dried. 10.32 g are obtained. Melting point is 209-212° C.

### Examination of acetylating properties

It is known that intact animals, receiving sufficient doses of pantothenic acid, have accelerated reactions of arylamine biotransformation. Therefore, there is the possibility to regulate the acetylating processes in the bodies of people and animals. In the examples given below the total acetylating capability of the organism was assessed using the method of quantitative determination of the arylamine content in the urine examined.

As a subject for acetylating, aromatic amines were chosen, as their usage makes it possible to assess both the activity of N-acetylamine transferase and (indirectly) the content of acetyl CoA.

### Example 3

As a subject for examination sodium sulfacyl was chosen. The examination of acetylating capability of white rats' was carried out using mongrel pubescent male rats for a 4-day period of administration of calcium ketopantothenate injections. 14 animals were included in each group. The examined agent was injected intraperitoneally at a dose of 20 mg/kg. On the 4^{th} day, 4 hours after the last injection of the examined agent, the sodium sulfacyl solution in a dose of 20 mg/kg was injected intraperitoneally. All the rats were separated into exchangeable cages for urine gathering. The collected data were compared with the control groups of animals (group I - only sodium sulfacyl in a dose of 20 mg/kg, and group II - calcium pantothenate).

The average weight of the animals in the experimental group fluctuates within 158.2 ± 12.3 g, correspondingly the absolute dose of sodium sulfacyl was 3.16 ± 0.25 mg. The changes of diuresis showed that the examined agent, like calcium D-pantothenate, did not change the volume of excreted urine in comparison with the control group I. Analysis of the results of the experiment shows that the percentage of the sulfacyl excreted with the urine did not differ significantly from the results of group I and II.

The claimed agent tripled the excretion of acetylated product in comparison with the control group. At the same time the excretion of free product was 10% lower. In comparison with calcium D-pantothenate the excretion of sulfacyl practically did not change, both in acetylated and in free form (Tables 1, 2).

Therefore, the results of the experiments showed that the total acetylating capability of the organism of white rats, according to the test of acetylation of aromatic amines (N-acetylation) increased significantly under the influence of calcium ketopantothenate. Also the activity of the novel agent was insignificantly higher than the activity of calcium D-pantothenate.

### Toxicity testing

### Example 4

To determine acute toxicity of calcium ketopantothenate 42 white non-linear mice of both sexes with the average weight of 18 ± 3 g were used in the experiment. The animals were distributed into 7 groups, 6 animals in each group. The first 6 groups received calcium ketopantothenate internally once in 0.2 ml of distilled water in doses: 3, 4, 5, 6, 7, 8 g/kg of the body weight, the 7th group was the control one, the mice of it received only 0.2 ml of distilled water internally once.

The mice were under observation for 14 days. Starting from the dose of 6g/kg in the first 2 hours after the agent administration the intoxication symptoms were observed: inertia, quickened breathing. Then these symptoms passed without consequences. The mice in all groups were practically healthy, no deviations in the state and behavior of the animals were observed.

When the experiment was over after the slaughtering and visual examination of the internal organs no changes were noticed.

It was not possible to increase the doses of the agent because of its further insolubility.

Calcium ketopantothenate may be considered as a low toxic compound, as its dose of 8.0 g/kg was not toxic, while the therapeutic dose of calcium D-pantothenate is 13.2 mg/kg and the LD₅₀ for calcium D-pantothenate administered perorally, is 5.92 g/kg. Therefore, calcium ketopantothenate may be considered less toxic than calcium D-pantothenate.

### Example 5

The study of subchronic toxicity of calcium ketopantothenate was carried out for 2 months in comparison with calcium D-pantothenate. 70 non-linear white rats of both sexes with average stated mass of 72 ± 5 g, divided into 7 groups, were used in the experiment. The first three groups of animals received ketopantothenate in 0.2 ml of distilled water in doses: 13.2 mg/kg (therapeutic dose of calcium D-pantothenate₎, 132 and 264 mg/kg (doses 10 and 20 times larger than the therapeutic dose) internally every day during the experiment. The next three groups of animals received the same doses of calcium D-pantothenate under the same conditions for comparison. The 7^{th} group was the control one and included intact animals. The weighing of the animals was carried out once a week. Permanent observation on development and state of rats was carried out. The growth of the animals' mass during the experiment is shown in Table 3, which shows that both calcium ketopantothenate and calcium D-pantothenate were not toxic agents when used over a prolonged period. The growth of the mass of the rats receiving ketopantothenate in different doses was 3.7 - 6.0 % higher than the growth of the mass of rats from the control group (correspondingly to doses). The growth of the mass of the rats receiving calcium D-pantothenate in comparison with the control group was approximately the same.

When the experiment was over the animals were slaughtering and the internal organs were examined visually. No irritation caused by ketopantothenate was observed in any of the groups.

The study of acute and subchronic toxicity of calcium ketopantothenate administered in different doses for a prolonged period and of local irritating effect caused by the agent administered for 2 months, showed that ketopantothenate in the acute experiment is less toxic than calcium D-pantothenate, and is a low toxic compound. When administered for a prolonged period in a therapeutic dose and in doses 10 and 20 times larger than the therapeutic dose, it was demonstrated to be a non-toxic agent with marked properties of calcium D-pantothenate. Internal doses given for a prolonged period do not have any local irritating effect.

Thus, the resulting data on acetylating properties and toxicity testify to the fact that calcium ketopantothenate has obvious acetylating properties and is a low toxic compound.

### Industrial applicability.

The results of the studies demonstrate that the novel compound, namely calcium ketopantothenate has vitamin activity, not less than the vitamin activity of calcium D-pantothenate, with considerably lower production cost and lower toxicity. It can be widely used in medicine and livestock breeding as a vitamin agent (particularly as an aqueous solution), or in the process of production of vitamin remedies containing the given compound. It can be also used as a vitamin additive to cosmetic products.

**Table 1**

| N | Group of animals, Agent | Sulfacyl dose, M± m | Diuresis M± m |
|---|---|---|---|
| 1 | Group I control (Na sulfacyl) | 3.13 ± 0.09 | 3.4 ± 0.18 |
| N | Group of animals, Agent | Sulfacyl dose, M± m | Diuresis M± m |
| 2 | Group II ( Ca D-pantothenate & Na sulfacyl) | 3.0 ± 0.08 | 3.9 ± 0.2 |
| 3 | Group III ( Ca ketopantothenate & Na sulfacyl) | 3.16 ± 0.25 | 3.3 ± 0.15 |

Continuation of table 1

| N | Na sulfacyl excretion | | | |
|---|---|---|---|---|
| | Sulfacyl isolation M ± m | | | Excretion % from the dose |
| | In free form | In acetylated form | Total | |
| 1 | 2.34 ± 0.13 | 0.28 ± 0.03 | 2.62 ± 0.17 | 83.9 |
| 2 | 2.12 ± 0.09 | 0.67 ± 0.04 | 2.79 ± 0.19 | 93.0 |
| 3 | 2.09 ± 0.17 | 0.73 ± 0.05 | 2.82 ± 0.19 | 89.2 |

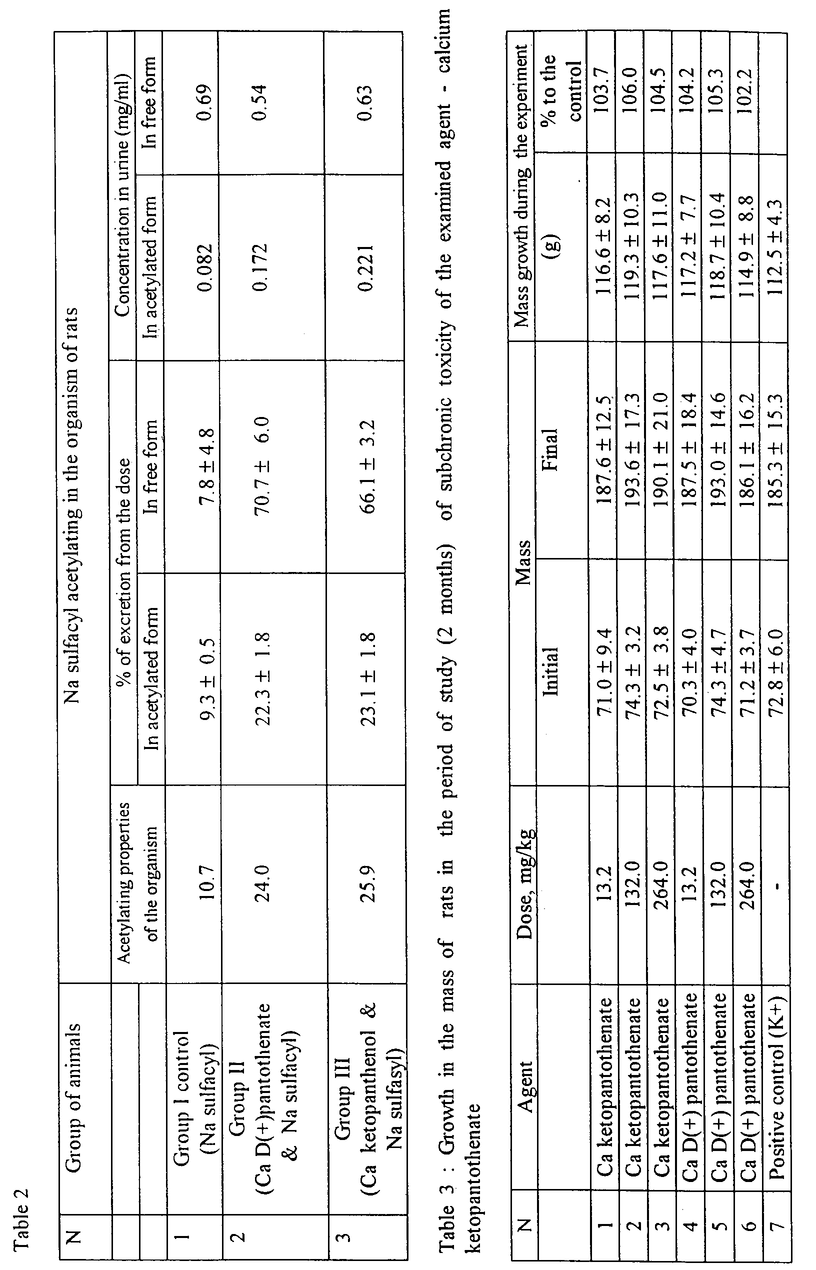

## Claims

1. Calcium ketopantothenate structure (1)

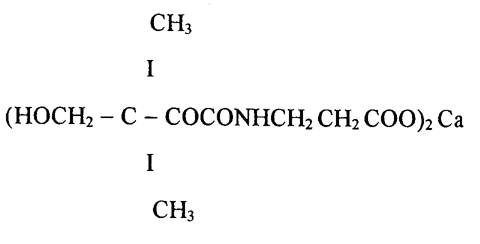

2. The method of obtaining of calcium ketopantothenate with structure (1) is characterized by the condensation of ketopantolactone with a calcium salt of β-alanine in low aliphatic alcohols as solvent at a temperature of 30-90° C, preferably at 65-70° C, and the product of the reaction is isolated by precipitation with acetone and evaporating and drying of the aqueous solution of the final product purified by extraction.

3. Calcium ketopantothenate with structure (1) demonstrating acetylating properties in the bodies of people and animals.

4. Usage of calcium ketopantothenate with structure (1) as a vitamin agent.

5. Usage of calcium ketopantothenate with structure (1) for the production of vitamin remedies.

6. Usage of calcium ketopantothenate with structure (1) as a vitamin additive to cosmetic products.
